# EUROPÄISCHE PATENTSCHRIFT

(11) **EP 1 231 861 B1**
(45) Veröffentlichungstag und Bekanntmachung des Hinweises auf die Patenterteilung: **06.07.2005**
(21) Anmeldenummer: 00985050.4
(22) Anmeldetag: 16.11.2000
(51) Int. Cl.: A61B 6/04

(54) **LAGERFLÄCHE FÜR EINEN MEDIZINISCHEN UNTERSUCHUNGSTISCH**
BEARING SURFACE FOR A MEDICAL EXAMINING-TABLE
SURFACE D'APPUI POUR UNE TABLE D'EXAMEN MEDICAL

(30) Priorität: 16.11.1999 DE 19955119
(43) Veröffentlichungstag der Anmeldung: 21.08.2002
(73) Patentinhaber: MAQUET GmbH & Co. KG, 76437 Rastatt (DE)
(72) Erfinder: PFEUFFER, Reinhard, 76477 Elchesheim-Illingen (DE); RÖDER, Siegfried, 76275 Ettlingen (DE); LICHT, Isolde, 77815 Bühl (DE); KATZENSTEIN, Bernhard, 79199 Kirchzarten (DE); BORNHEIMER, Heiko, 65205 Wiesbaden (DE)
(74) Vertreter: Schaumburg, Thoenes, Thurn, Landskron
(86) Internationale Anmeldenummer: PCT/EP2000/011394
(87) Internationale Veröffentlichungsnummer: WO 2001/035828

(56) Entgegenhaltungen:
- EP-A- 0 899 026
- EP-A- 0 923 922
- DE-A- 1 566 126
- DE-A- 2 613 863
- US-A- 4 475 072
- US-A- 4 700 938

## Beschreibung

Die Erfindung betrifft eine Lagerfläche für einen medizinischen Untersuchungstisch zur Durchführung röntgenologischer Untersuchungen und zur Durchführung chirurgischer Eingriffe mit intra-operativem Einsatz von Röntgengeräten, umfassend eine Tischplatte und eine mit einer Tragsäule des Untersuchungstisches koppelbare Führungseinheit mit einem ersten Führungsgehäuse, in dem die Tischplatte in ihrer Längsrichtung verschiebbar geführt ist.

Spezielle Untersuchungen und chirurgische Eingriffe (zum Beispiel in der Gefäßchirurgie) erfordern eine Relativbewegung zwischen dem Röntgengerät und dem Untersuchungsfeld, da sich dieses über einen relativ großen Bereich erstrecken kann, der größer als das statische Bildfeld des Röntgengerätes ist. Eine Verstellung des Röntgengerätes ist nur bedingt möglich, so daß es erforderlich ist, die Lagerfläche des Untersuchungstisches mit dem Patienten zu verschieben. Um einen ungehinderten Einsatz des Röntgengerätes zu ermöglichen, darf sich unterhalb des Untersuchungsfeldes kein störendes Hindernis befinden. Das bedeutet, daß die Lagerfläche weit über die Tragsäule des Untersuchungstisches hinausragen muß.

Lagerflächen der eingangs genannten Art für röntgenologische Untersuchungen, zum Beispiel mit Herzkathetem, sind bekannt. Bei diesen Lagerflächen sind die Tischplatten aber mit Metallschienen versehen, die zur Längsverschiebung der Tischplatten in speziellen Führungselementen an den Führungseinheiten geführt werden. Diese Führungsschienen machen sich im Röntgenbild als Schatten bemerkbar, hinter dem keine Körperstrukturen des Patienten mehr erkennbar sind. Der Bereich, in dem sich diese Führungschienen befinden, ist also nicht artefaktfrei durchstrahlbar.

Die Länge der Führungsschienen ergibt sich aus der Größe der Führungslänge, die erforderlich ist, um die aus dem Patientengewicht resultierenden Drehmomente abzustützen, und aus dem erforderlichen Hub für die Längsverschiebung. Daraus folgt, daß der nicht frei durchstrahlbare Bereich einer solchen Lagerfläche im Vergleich zur Größe des zu untersuchenden Untersuchungsfeldes sehr groß ist. Die gesamte Länge der Lagerfläche setzt sich dabei zusammen aus der Länge der Führungsschienen und der Länge des rundum durchstrahlbaren "Nutzbereichs", so daß sich eine große Längsausdehnung des Systems ergibt.

Eine Lagerfläche der eingangs genannten Art ist aus der US-A-4,700,938 bekannt. Die dort beschriebene Lagerfläche besteht aus einem Schaumstoffkern und einer Außenhülle aus kohlefaserverstärktem Kunststoff. Die Außenhülle besteht aus einem Oberteil, einem Unterteil und zwei C-Profilen, welche sich entlang den Längsrändern der Tischplatte erstrecken und an ihren C-Schenkeln mit dem Oberteil und dem Unterteil verbunden sind. Die Tischplatte liegt auf Rollen auf, die in einer Tragsäule gelagert sind. An der Tragsäule sind ferner Spurrollen drehbar gelagert, die in das jeweilige C-Profil von außen her eingreifen und auf dem unteren C-Schenkel des C-Profils aufliegen, der zusammen mit dem Unterteil der Außenhülle der Tischplatte einen doppellagigen Randstreifen bildet. Der obere C-Schenkel des C-Profils ist zusammen mit einem Abschnitt des Oberteils der Außenhülle über die jeweilige Spurrolle nach außen gezogen und ein Stück nach unten abgewinkelt, so daß dieser Abschnitt eine Abdeckung für die Spurrollen bildet.

Der vorliegenden Erfindung liegt die Aufgabe zugrunde, eine mindestens längsverschiebbare Patientenlagerfläche der eingangs genannten Art anzugeben, die bei insgesamt kurzer Baulänge einen großen mit Röntgenstrahlen frei durchstrahlbaren Bereich hat und einen großen Verschiebeweg in Längsrichtung ermöglicht.

Diese Aufgabe wird erfindungsgemäß durch die im Anspruch 1 angegebenen Merkmale gelöst.

Bei der erfindungsgemäßen Lagerfläche ist der nicht durchstrahlbare Bereich des Untersuchungstisches also auf den Bereich der Führungseinheit beschränkt, der für das Röntgengerät ohnehin nicht zugänglich ist, da sich unterhalb der Führungseinheit die Tragsäule des Untersuchungstisches befindet. Außerhalb des Führungsgehäuses dagegen ist die Tischplatte artefaktefrei durchstrahlbar, da sie selbst keinerlei Metallteile trägt, welche das Röntgenbild beeinträchtigen könnten. Das Fehlen metallischer Führungsschienen oder sonstiger Führungselemente ist bebonders vorteilhaft, wenn zur optimalen Darstellung der zu untersuchenden Organe das Röntgengerät mit unterschiedlichen Projektionsrichtungen eingesetzt wird. Die Tischplatte kann also auch von der Seite oder schräg von unten durchstrahlt werden, ohne daß Schatten der abgebildeten Metallteile die Körperstrukturen des Patienten verdecken.

Vorzugsweise ist die Tischplatte aus kohlefaserverstärktem Kunststoff hergestellt, da dieses Material den Vorteil einer guten Röntgenstrahlendurchlässigkeit bei gleichzeitig hohen Festigkeitswerten besitzt.

Bei einer bevorzugten Ausführungsform hat die Tischplatte zwei in Längsrichtung verlaufende Randstreifen und einen gegenüber den Randstreifen wesentlich dickeren Mittelabschnitt. Dieser Mittelabschnitt kann quer zu seiner Längsrichtung einen trapezförmigen Querschnitt haben, wobei die Unterseite des Mittelabschnittes schmaler als die Oberseite ist, so daß die Platte sich in ihrem Mittelabschnitt schräg nach unten verjüngt. Durch die höhere Dicke des Mittelabschnittes ergibt sich eine höhere Belastbarkeit der Platte, wobei die schräggerichteten Seitenflächen der Tischplatte dafür sorgen, daß der Hell-Dunkel-Übergang am Plattenrand im Röntgenbild nicht so schroff ausfällt.

Vorzugsweise sind die Führungselemente obere und untere Stütz- oder Führungsrollen, die gleichzeitig direkt an oberen beziehungsweise unteren Führungsflächen der Tischplatte anliegen. Die oberen und unteren Führungsflächen können dabei entlang den beiden Längsrändern der Tischplatte auf der Oberseite beziehungsweise Unterseite derselben ausgebildet sein. Beispielsweise können die oberen und unteren Führungsflächen an den vorstehend genannten Randleisten beiderseits des Mittelabschnittes der Tischplatte ausgebildet sein.

Die oberen und unteren Führungsrollen können jeweils zu einem Rollenblock zusammengefaßt und an einem Rollenträger gelagert sein, der um eine quer zur Längsrichtung und parallel zur Tischplatte gerichtete Achse an dem Führungsgehäuse schwenkbar gelagert ist, wobei auf jeder Längsseite der Tischplatte mindestens zwei Rollenblöcke an dem Führungsgehäuse angeordnet sind. Durch das Vorsehen mehrerer Rollen und das Zusammenfassen der Rollen zu Rollenblöcken erreicht man eine gleichmäßige Verteilung der Last auf die Rollen. Die Rollen bestehen vorzugsweise aus Kunststoff und sind zur Verminderung des Rollwiderstandes vorzugsweise mit Wälzlagern gelagert. Durch die schwenkbare Lagerung des Rollenträgers kann sich der jeweilige Rollenblock bei einer Durchbiegung der Tischplatte an die Verformung der Randleisten der Tischplatte anpassen, wobei sich ebenfalls eine gleichmäßige Verteilung der Kräfte auf alle Rollen ergibt. Bei einer abgewandelten Ausführungsform können die oberen Führungsflächen an den Randstreifen der Tischplatte ausgebildet sein, während an der Unterseite des Mittelabschnittes mindestens eine untere Führungsfläche vorgesehen ist. Dies bietet die Möglichkeit, den Mittelabschnitt der Tischplatte abzustützen, indem sich die an der mindestens einen unteren Führungsfläche anliegende Führungsrolle quer über die gesamte Breite der Tischplatte erstreckt.

Die Führungseinheit kann ein zweites Führungsgehäuse haben, an dem Kopplungs- und Führungselemente für die Kopplung mit der Tragsäule und eine Querbewegung der Lagerfläche relativ zur Tragsäule vorgesehen sind. Durch die lösbare Verbindung zwischen der Führungseinheit und der Tragsäule können die Lagerflächen ausgetauscht und somit spezielle Lagerflächen für spezielle Untersuchungen oder Operationsdisziplinen auf dem gleichen Sockel eingesetzt werden. Zusätzlich zu der Längs- und Querverschiebung der Tischplatte können auch Möglichkeiten vorgesehen sein, um die Lagerfläche in der Höhe zu verstellen und in Längs -und/oder Querrichtung zu neigen. Üblicherweise sind die Mittel für diese Verstellungen in der Tragsäule vorgesehen.

Um zu vermeiden, daß bei einer Neigung der Lagerfläche in Längsrichtung eine unbeabsichtigte Verschiebung der Lagefläche aufgrund der aus dem Patientengewicht resultierenden Hangabtriebskraft auftritt, ist in dem Führungsgehäuse zweckmäßigerweise mindestens eine Bremseinheit angeordnet, die zur Anlage an Flächen der Tischplatte, z.B. an den oberen und/oder unteren Führungsflächen bestimmte Bremselemente und einen Stellmechanismus für die Bremselemente hat. Vorzugsweise sind Bremseinheiten auf beiden Seiten der Führung angeordnet, um eine erhöhte Bremswirkung zu erzielen, Redundanz zu erzeugen und eine ungleichmäßige Krafteinleitung zu vermeiden. Die Bremselemente können von zwei Bremsbacken gebildet sein, die auch auf die Ober- und Unterseite der Randleisten der Tischplatte einwirken und mittels eines Elektromagneten gelüftet werden können. Aus Sicherheitsgründen ist es zweckmäßig, die Bremse im stromlosen Zusand aktiv, das heißt die Bremsbacken geschlossen zu halten. Die Bremskraft wird in diesem Fall durch eine vorgespannte Feder erzeugt. Zum Öffnen der Bremse wird der Elektromagnet erregt und lüftet die Bremsbacken gegen die Federkraft, so daß eine Verschiebung der Lagerfläche in Längsrichtung erfolgen kann. Um zu verhindern, daß sich bei der sich aufgrund der Belastung der Lagerfläche ergebenden Durchbiegung ein Kontakt der Führungsflächen mit den Bremsbacken ergibt, sind die Bremselemente und der Verstellmechanismus zweckmäßigerweise an einen Bremsenträger angeordnet, der mittels Rollen an einer oberen Führungsfläche der Tischplatte gelagert ist und gegen eine Bewegung in Längsrichtung an dem oberen Führungsgehäuse gesichert ist. Die gesamte Bremseinheit ist also an der Tischplatte mittels Rollen schwimmend gelagert und stützt sich nur in Längsrichtung am Rahmen des ersten Führungsgehäuses ab.

Die Verschiebung der Tischplatte kann manuell oder motorisch erfolgen. Vor einer Verschiebung müssen zunächst die Bremsen gelüftet werden. Dies kann entweder über einen Fußschalter oder über einen manuell zu betätigenden Schalter an der Lagerfläche erfolgen. Aus Sicherheitsgründen soll ein Lüften der Bremsen nur dann ermöglicht werden, wenn die Lagerfläche horizontal ausgerichtet ist, um zu verhindern, daß die Lagerfläche bei einer Längs- oder Quemeigung eine unkontrollierte Bewegung ausführt. In diesem Falle ist also ein Steuerung vorzusehen, die in Abhängigkeit der Winkelstellung des Kopfes der Tragsäule, durch welche die Höhenverstellung, Neigung in Längsrichtung und die Kantung in Querrichtung realisiert wird, die Betätigung der Bremsen freigibt oder unterdrückt.

Zur motorischen Verstellung der Lagerfläche kann mindestens ein Friktionsantrieb in dem Führungsgehäuse vorgesehen sein, der an einem Randstreifen der Tischplatte angreift. Der Friktionsantrieb kann beispielsweise eine mit einem Motor gekoppelte Antriebsrolle und zwei Reibrollen umfassen, die einerseits in permanentem Eingriff mit der Antriebsrolle stehen und andererseits gegen eine Führungsfläche der Tischplatte gespannt sind. Der Motor ist vorzugsweise ein Elektromotor. Mit der erfindungsgemäßen Lösung wird vermieden, daß zur Umsetzung der Drehbewegung des Motors in eine Linearbewegung der Tischplatte Elemente wie Zahnstangen, Gewindespindeln oder Zugmittel verwendet werden müssen, die den Nachteil haben, daß sie zur Bewegungsumwandlung teilweise im Strahlengang des Röntgengerätes liegen müßten, um den großen Hub zu erzeugen. Dadurch würden im Bildfeld Schatten entstehen, welche die Überwachung beziehungsweise Untersuchung der Körperstrukturen erschweren. Dadurch daß die Reibrollen beweglich gelagert und gegen die Führungsbahn sowie die Antriebsrolle gespannt werden, kann ein Verschleiß ausgeglichen und Verformungen der Lagerfläche durch ihre Belastung Rechnung getragen werden.

Zur Erzielung einer ausreichend großen Vortriebskraft muß man den Reibwert der Antriebsrollen durch die Wahl eines geeigneten Werkstoffes wie zum Beispiel Gummi oder Polyurethan vergrößern. Da diese Werkstoffe keine besonders große zulässige Flächenpressung haben, ist die erreichbare Anpreßkraft und damit auch die Reibkraft bei gegebener Rollenbreite begrenzt. Durch die Verbreiterung der Reibrollen kann die für den Reibwerkstoff zulässige Anpreßkraft soweit vergrößert werden, daß diese Antriebsrollen gleichzeitig auch die Funktion der Abstützung der von der Platte auf die Führungseinheit einwirkenden Gewichtskräfte übernehmen können. Die erreichbare Vortriebskraft ist in diesem Falle direkt von der Belastung der Rollen abhängig, was den Vorteil hat, daß auch bei großen Patientengewichten und bei großer Ausladung der Lagerfläche über die Führung hinaus eine ausreichend große Vortriebskraft erzeugt wird. Durch die Kombination der beiden Funktionen "Führen" und "Antreiben" ergibt sich somit auch ein einfacher Aufbau der Längsführung für die Lagerfläche. Dieser Vorteil läßt sich insbesondere bei der oben beschriebenen Lösung erreichen, bei welcher die untere Führungsrolle sich quer über die gesamte Breite der Tischplatte erstreckt, indem man diese Führungsrolle mit einem Antriebsmotor koppelt.

Um die gewünschte Verschiebung der Lagerfläche in Querrichtung zu realisieren, ist das obere oder erste Führungsgehäuse mittels geeigneter Führungselemente gegenüber dem unteren oder zweiten Führungsgehäuse oder dieses gegenüber der Tragsäule verschiebbar angeordnet. Dabei ist es für die erforderliche Querverschiebung der Lagerfläche nicht notwendig, durchleuchtbare Führungen zu realisieren, da sich das untere Führungsgehäuse im Bereich der Tragsäule befindet, wo ohnehin keine Zugangsmöglichkeit für das Röntgengerät besteht. Daher können hier bekannte Führungselemente eingesetzt werden. Da die Lagerfläche auch seitlich geneigt oder gekantet werden kann, ist es zweckmäßig, auch für die Querführung eine Bremseinrichtung vorzusehen, um eine unbeabsichtigte Verschiebung der Tischplatte zu vermeiden.

Weiter Merkmale und Vorteile der Erfindung ergeben sich aus der folgenden Beschreibung, welche in Verbindung mit den beigefügten Zeichnungen die Erfindung anhand von Ausführungsbeispielen erläutert. Es zeigen:
- Fig. 1: eine schematische perspektivische Gesamtansicht eines Untersuchungstisches mit der erfindungsgemäßen Lagerfläche in zwei verschiedenen Stellungen,
- Fig. 2: einen schematischen Längsschnitt durch einen Seitenholm des oberen Führungsgehäuses,
- Fig. 3: einen vergrößerten Längsschnitt durch einen Rollenblock der Führung allein,
- Fig.4: einen Schnitt quer zur Längsrichtung durch einen Seitenholm des oberen Führungsgehäuses und den Randbereich der Tischplatte entlang Linie IV-IV in Figur 2,
- Fig. 5: eine schematische, teilweise geschnittene Seitenansicht einer Bremseinheit,
- Fig. 6: eine schematische, teilweise geschnittene Seitenansicht einer Antriebseinheit für eine Längsverschiebung der Tischplatte
- Fig. 7: eine schematische Seitenansicht einer abgewandelten Ausführungsform der erfindungsgemäßen Lagerfläche mit kombinierten Antriebs- und Führungsrollen und
- Fig. 8: einen Schnitt quer zur Längsrichtung durch die zweite Ausführungsform der erfindungsgemäßen Lagerplatte entlang der Linie VIII-VIII in Figur 7.

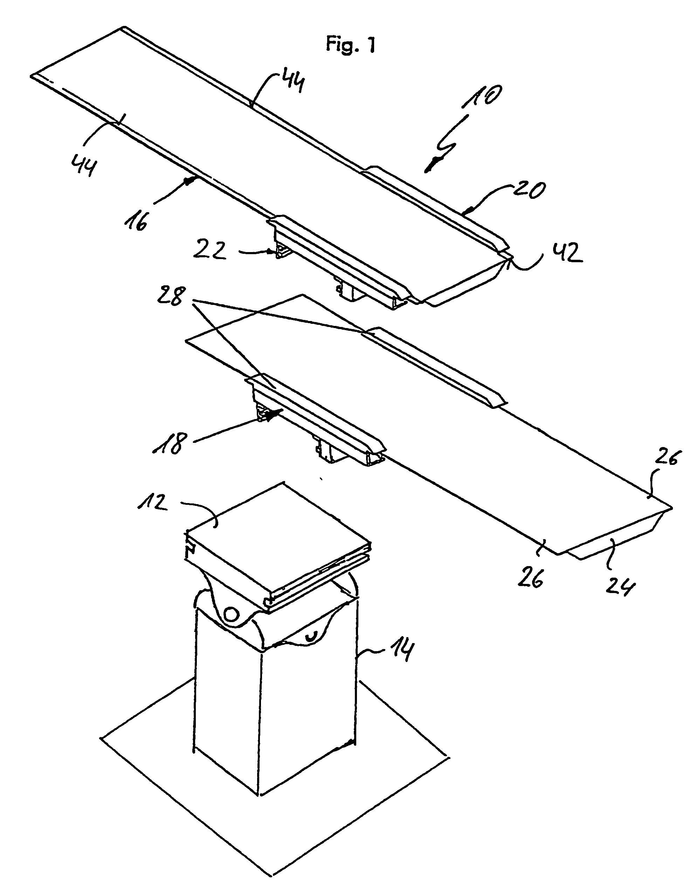

Figur 1 zeigt eine Gesamtansicht einer Patientenlagerfläche 10, die mit dem Kopf 12 einer Tragesäule 14 eines Untersuchungstisches verbindbar ist. Die Lagerfläche 10 besteht aus einer Tischplatte 16 und einer Führungseinheit 18, die ein oberes Führungsgehäuse 20 und ein unteres Führungsgehäuse 22 umfaßt. Die Tischplatte 16 ist in dem oberen Führungsgehäuse 20 in ihrer Längsrichtung verschiebbar geführt, wie dies durch die beiden Stellungen der Tischplatte 16 in Figur 1 dargestellt ist. Das untere Führungsgehäuse 22 ist an dem Kopf der Tragsäule 12 in nicht dargestellter Weise verschiebbar geführt, so daß die Lagerfläche 10 insgesamt quer zur Längsrichtung der Tischplatte 16 verschiebbar ist.

Die Tischplatte 16 hat einen dickeren Mittelabschnitt 24 mit trapezförmigem Querschnitt und zwei dünnen Randstreifen 26 entlang ihren Längsseiten. Die Tischplatte 16 ist insgesamt, und zwar auch im Bereich der beiden Randstreifen 26 ohne störende Metallteile hergestellt und deswegen artefaktefrei durchleuchtbar. Beispielsweise besteht die Tischplatte 16 aus kohlefaserverstärktem Kunststoff.

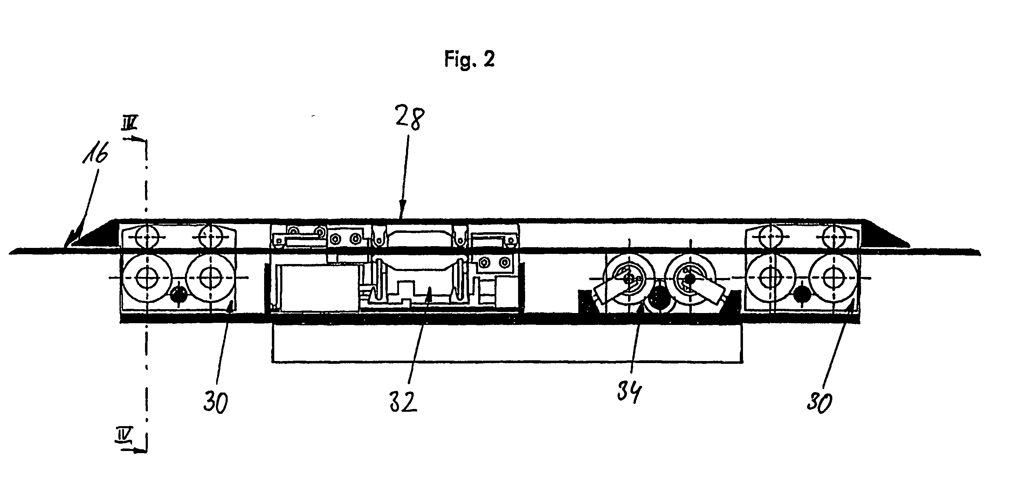

Figur 2 zeigt einen Seitenholm 28 des oberen Führungsgehäuses 20. An den Enden des Seitenholms 28 befinden sich zwei Rollenblöcke 30, die der Führung der Tischplatte 16 in Längsrichtung dienen. Zwischen den Rollenblöcken 30 ist auf der einen Seite eine Bremseinheit 32 angeordnet, die senkrecht zur Ebene der Tischplatte 16 frei verschiebbar ist und sich in Längsrichtung am Holm 28 abstützt. Ferner befindet sich zwischen den beiden Rollenblöcken 30 eine Antriebseinheit 34 zum Verschieben der Tischplatte 16.

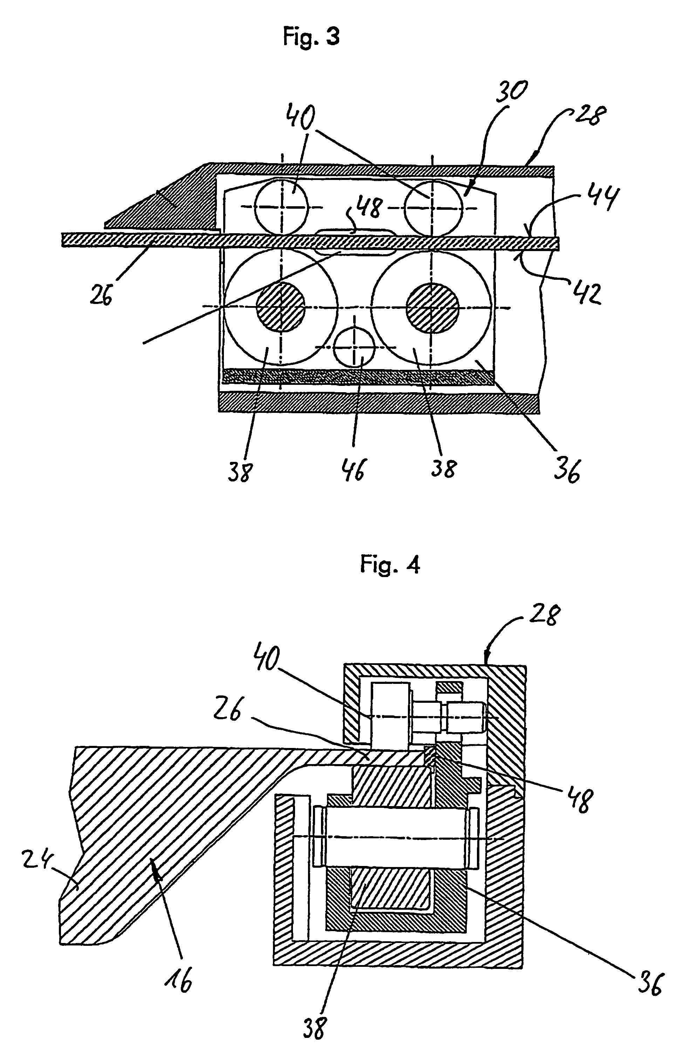

In den Figuren 3 und 4 ist ein Rollenblock 30 näher dargestellt. Der Rollenblock besteht aus einem Rollenträger 36, in dem untere Stützrollen 38 und obere Stützrollen 40 gelagert sind. Zwischen den Rollen 38 und 40 liegt ein Randstreifen 26 der Tischplatte 16, so daß die unteren Rollen 38 und die oberen Rollen 40 auf einer unteren Führungsfläche 42 beziehungsweise einer oberen Führungsfläche 44 laufen, welche von der Unter- beziehungsweise Oberseite des Randstreifens 26 gebildet sind. Zur Realisierung einer gleichmäßigen Lastverteilung ist der Rollenträger 36 um eine Achse 46 schwenkbar an dem Seitenholm 28 gelagert. Bei einer Längsverschiebung der Tischplatte 16 wälzen sich die Rollen 38 und 40 auf dem Randstreifen 26 der Tischplatte 16 ab. Biegt sich die Tischplatte aufgrund der Belastung durch, so stellt sich der Rollenblock 30 durch eine Schwenkbewegung um die Achse 46 so ein, daß auf beide Rollenpaare38, 40 ungefähr die gleiche Kraft einwirkt. Dadurch verringert sich sowohl die Belastung der Rollen als auch die Belastung der Oberfläche der Tischplatte 16, was zu einer Reduktion des Verschleißes an der Tischplatte 16 führt. Zur seitlichen Führung der Tischplatte 16 sind in dem Rollenträger 36 Führungsleisten 48 angebracht, die ein seitliches Ausweichen der Tischplatte 16 relativ zum oberen Führungsgehäuse 20 verhindern.

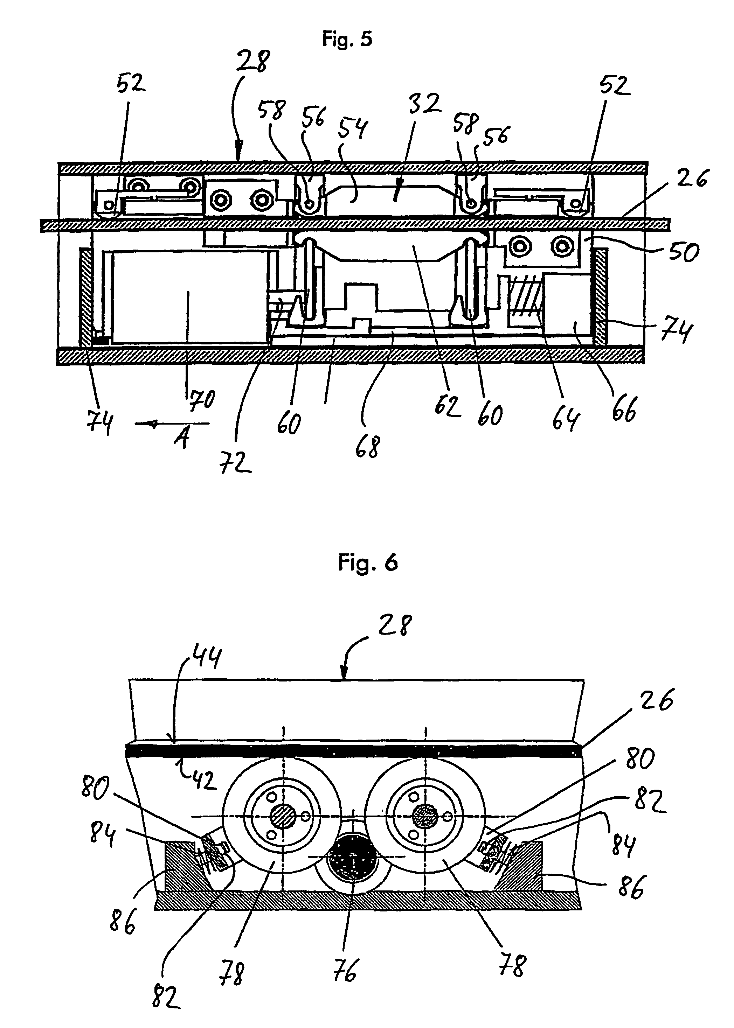

Figur 5 zeigt die Bremseinheit 32 im vergrößerten Maßstab. Die Bremseinheit dient dazu, eine unbeabsichtigte Bewegung der Tischplatte 16 aufgrund äußerer Einflüsse zu vermeiden. Die Bremseinheit umfaßt einen Rahmen 50, an dem Rollen 52 gelagert sind, mit dem die gesamte Bremseinheit 32 auf der Randleiste 26 der Tischplatte 16, das heißt auf der oberen Führungsfläche 44 einer Randleiste 26 läuft. An dem Rahmen 50 ist ein oberer Bremsschuh 54 befestigt. An dem oberen Bremsschuh 54 sind zwei Hebel 56 um jeweils eine Achse 58 schwenkbar gelagert, die über weitere Hebel 60 einen unteren Bremsschuh 62 tragen. Werden die beiden Hebel 56 um ihre Achsen 58 verschwenkt, so ändert sich der Abstand zwischen den beiden Bremsschuhen 54 und 62. Die Anpreßkraft für die Bremsschuhe 54 und 62 wird durch eine Feder 64 erzeugt, die sich einerseits an einem rahmenfesten Teil 66 und andererseits an einem beweglichen Schieber 68 abstützt, der seinerseits auf die Hebel 56 einwirkt und diese in Figur 5 in Richtung des Pfeiles A nach links vorspannt. Die Kraft der Feder 64 wird durch die Übersetzung der Hebel verstärkt.

Zum Öffnen der Bremse dient ein Elektromagnet 70, der an dem Rahmen 50 befestigt ist und mit seinem Anker 72 auf einen der Hebel 60 einwirkt. Wird der Elektromagnet bestromt, wird der Anker 72 in Figur 5 entgegen der Richtung des Pfeiles A in Figur 5 bewegt. Die Magnetkraft kompensiert die Kraft der Feder 64, so daß der Schieber 68 in Figur 5 nach rechts verstellt und die Bremse gelüftet wird. Bei Bestromung des Elektromagneten 70 läßt sich somit die Tischplatte verschieben, während in stromlosem Zustand des Elektromagneten 70 die Bremse die Tischplatte 16 festhält. Da aufgrund der Hebelübersetzung zur Erzeugung einer großen Bremskraft der Relativhub zwischen dem oberen Bremsschuh 54 und dem unteren Bremsschuh 62 klein ist, muß verhindert werden, daß bei einer Durchbiegung der Tischplatte 16 diese an einem der Bremsschuhe schleift, wenn die Bremse gelüftet ist. Aus diesem Grunde ist die Bremseinheit 32 mittels der Rollen 52 auf dem Randstreifen 26 der Tischplatte 16 geführt, so daß die Bremsschuhe 54 und 62 unabhängig von der Durchbiegung der Tischplatte 16 stets den gleichen Abstand von der Plattenoberfläche haben. In Längsrichtung stützt sich die Bremseinheit 32 an Zwischenwänden 74 des Seitenholms 28 ab.

Figur 6 zeigt die Antriebseinheit 34, die zum Verschieben der Tischplatte 16 in ihrer Längsrichtung dient. Sie umfaßt eine Antriebsrolle 76, die von einem nicht dargestellten Elektromotor angetrieben wird. Die Antriebsrolle 76 steht in Kontakt mit zwei Reibrollen 78, die an der unteren Führungsfläche 42 eines Randstreifens 26 der Tischplatte 16 anliegen. Die Reibrollen 78 sind jeweils an einem Rollenträger 80 gelagert, der auf einem Bolzen 82 innerhalb gewisser Grenzen frei verschieblich ist, so daß eine Veränderung des Abstandes zwischen der Antriebsrolle 76 und der Tischplatte 16 aufgrund der Belastung der Lagerfläche oder aufgrund von Verschleiß durch die Verstellbarkeit der Reibrollen 78 ausgeglichen werden kann. Die erforderliche Anpreßkraft für die Übertragung eines Moments durch Reibung wird jeweils durch eine Feder 84 erzeugt, die sich an einem den Bolzen 82 haltenden Bock 86 abstützt und die Reibrolle 78 über den Rollenträger 80 gegen die Antriebsrolle 76 einerseits und die untere Führungsfläche der Randleiste 26 andererseits spannt.

Die Bremseinheit 32 und die Antriebseinheit 34 können nur in einem der Seitenholme 28 oder auch in beiden Seitenholmen des oberen Führungsgehäuses 20 vorgesehen sein.

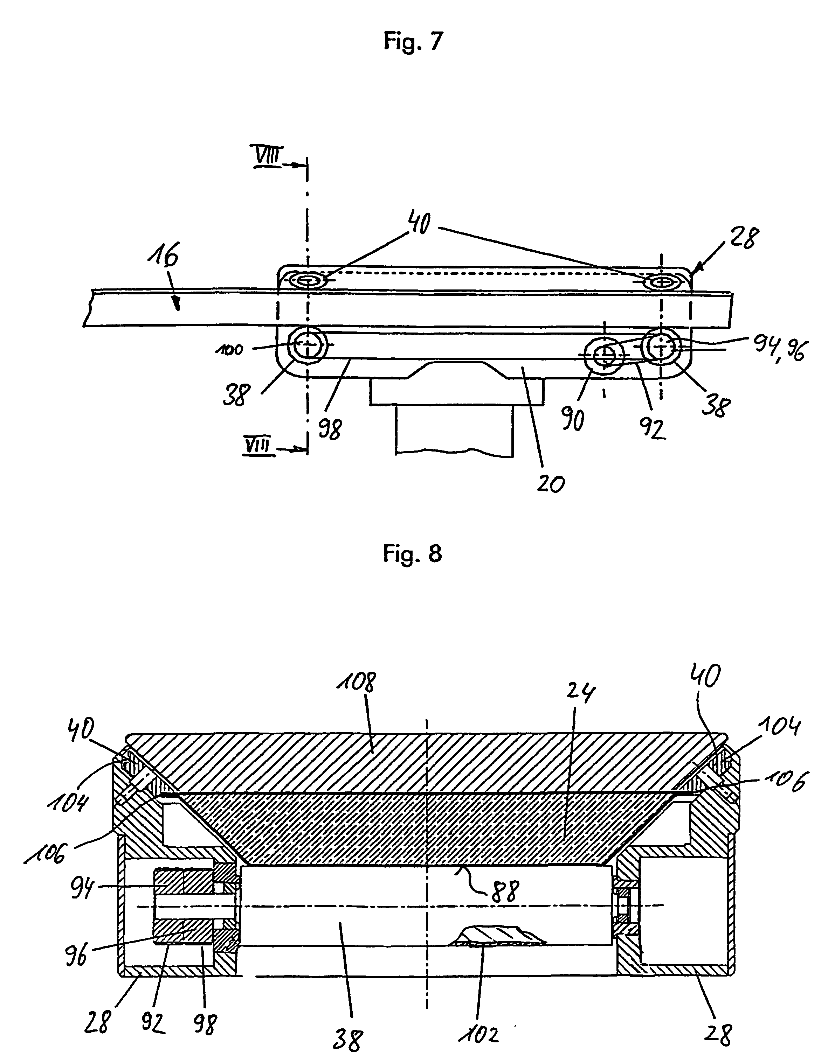

Die Figuren 7 und 8 zeigen eine zweite Ausführungsform der Erfindung, wobei gleiche Teile wieder mit gleichen Bezugszeichen versehen sind. Bei der in den Figuren 7 und 8 dargestellten Ausführungsform erstrecken sich die unteren Stützrollen 38 über die gesamte Breite des oberen Führungsgehäuses 20 und sind in den beiden Seitenholmen 28 gelagert. Damit liegt die Tischplatte 16 mit der als untere Führungsfläche 88 ausgebildeten Unterseite ihres Mittelabschnittes 24 auf den unteren Führungsrollen 38 auf. Dadurch wird die Flächenpressung sowohl an den Rollen als auch an der Tischplatte reduziert.

Bei der Ausführungsform gemäß den Figuren 7 und 8 dienen die unteren Stützrollen 38 gleichzeitig zum Antrieb für eine Verschiebung der Tischplatte 16. Ein Motor 90 treibt über einen Zahnriemen 92 und eine Riemenscheibe 94 eine der Stützrollen 38 an. Eine co-axial zu der Riemenscheibe 94 angeordnete zweite Riemenscheibe 96 überträgt die Drehbewegung der einen Stützrolle 38 mittels eines Zahnriemens 98 und einer Riemenscheibe 100 auf die andere Stützrolle 38. Dadurch wird sichergestellt, daß unabhängig von der Schwerpunktlage der Tischplatte 16 mit dem daraufliegenden Patienten eine konstante Vortriebskraft erzeugt wird. Die unteren Stützrollen 38 sind mit einem Belag 102 beschichtet, der einen hohen Reibkoeffizienten aufweist, um eine ausreichend hohe Vortriebskraft zu erzeugen.

Die oberen Stützrollen 40 sind bei der Ausführungsform gemäß Figur 8 schräggestellt, um die Höhe der Seitenholme 28 und damit auch die Gesamthöhe des Untersuchungstisches zu reduzieren. Die Rollen 40 sind bei dieser Ausführungsform als Spurrollen ausgeführt, die eine Umfangsnut 104 haben, so daß sie nicht nur auf der oberen Führungsfläche 44 des Randstreifens 26 aufliegen, sondern auch an der seitlichen Randfläche 106 der Tischplatte 16. Somit übernehmen die Rollen 40 bei dieser Ausführungsform zusätzlich zur Abstützung der Vertikalkräfte auch die seitliche Führung der Tischplatte 16.

Auf der Tischplatte 16 ist bei der Ausführungsform gemäß Figur 8 ein Polster 108 dargestellt, das den Liegekomfort der Patienten verbessern soll.

## Patentansprüche

1. Lagerfläche für einen medizinischen Untersuchungstisch zur Durchführung röntgenologischer Untersuchungen und chirurgischer Eingriffe mit mtraoperativem Einsatz von Röntgengeräten, mit einer Tischplatte (16), die vollständig aus röntgenstrahlendurchlässigem Material besteht und Führungsflächen (42, 44; 88) hat, mit denen sie an eine Verschiebung relativ zu einer Tragsäule (14) des Untersuchungstisches ermöglichenden Führungselementen (38, 40) anliegt, wobei die Tischerplatte (16) zwei ihre Längsränder bildende Randstreifen (26) sowie einen gegenüber den Randstreifen (26) dickeren Mittelabschnitt (24) hat, **dadurch gekennzeichnet, dass** die Lagerfläche (10) eine mit der Tragsäule (14) des Untersuchungstisches koppelbare Führungseinheit (18) mit einem ersten Führungsgehäuse (20) hat, in dem die Führungselemente (38, 40) angeordnet sind und in dem die Tischplatte (16) in ihrer Längsrichtung verschiebbar geführt ist, und daß mindestens die oberen Führungsflächen (44) auf der Oberseite der Randstreifen (26) ausgebildet sind.

2. Lagerfläche nach Anspruch 1, **dadurch gekennzeichnet, daß** der Mittelabschnitt (24) quer zu seiner Längsrichtung einen trapezförmigen Querschnitt hat, wobei die Unterseite des Mittelabschnittes (24) schmaler als dessen Oberseite ist

3. Lagerfäche nach Anspruch 1 oder 2, **dadurch gekennzeichnet, daß** die Führungselemente obere und untere Stütz- oder Führungsrollen (40, 38) sind, die gleichzeitig direkt an oberen beziehungsweise unteren Führungsflächen (44, 42) der Tischplatte (16) anliegen.

4. Lagerfläche nach einem der Ansprüche 1 bis 3, **dadurch gekennzeichnet, daß** die unteren Führungsflächen (44, 42) entlang den beiden Längsrändern (26) der Tischplatte (16) auf deren Unterseite ausgebildet sind.

5. Lagerfläche nach Anspruch 3 oder 4, **dadurch gekennzeichnet, daß** mehrere untere und obere Führungsrollen (38, 40) jeweils zu einem Rollenblock (30) zusammengefaßt und an einem Rollenträger (36) gelagert sind, der um eine quer zur Längsrichtung und parallel zur Tischplatte (16) gerichtete Achse (46) an dem Führungsgehäuse (20) schwenkbar gelagert ist, wobei auf jeder Längsseite der Lagerfläche (10) mindestens zwei Rollenblöcke (30) an dem Führungsgehäuse (20) angeordnet sind.

6. Lagerfläche nach einem der Ansprüche 1 bis 5, **dadurch gekennzeichnet, daß** in dem Führungsgehäuse (20) mindestens ein an einem Randstreifen (26) der Tischplatte (16) angreifender Friktionsantrieb (32) vorgesehen ist.

7. Lagerfläche nach Anspruch 6, **dadurch gekennzeichnet, daß** der Friktionsantrieb (32) eine mit einem Motor gekoppelte Antriebsrolle (76) und zwei Reibrollen (78) umfaßt, die einerseits im permanenten Eingriff mit der Antriebsrolle (76) stehen und andererseits gegen eine Führungsfläche (42) der Tischplatte (16) gespannt sind.

8. Lagerfläche nach einem der Ansprüche 1 bis 3, **dadurch gekennzeichnet, daß** die oberen Führungsftächen (44) an den Randstreifen (26) ausgebildet sind und daß an der Unterseite des Mittelabschnittes (24) mindestens eine untere Führungsfläche (88) ausgebildet ist.

9. Lagerfläche nach Anspruch 8, **dadurch gekennzeichnet, daß** sich die an der mindestens einen unteren Führungsfläche (88) anliegende Führungsrolle (38) quer über die gesamte Breite der Tischplatte (16) erstreckt.

10. Lagerfläche nach Anspruch 8 oder 9, **dadurch gekennzeichnet, daß** die oberen Führungsrollen (40) als Spurrollen mit einer Seitenführung (104) für die Tischplatte (16) ausgebildet sind.

11. Lagerfläche nach einem der Ansprüche 8 bis 10, **dadurch gekennzeichnet, daß** die unteren Führungsrollen (38) durch einen Motor (90) antreibbar sind.

12. Lagerfläche nach einem der Ansprüche 1 bis 11, **dadurch gekennzeichnet, daß** in dem Führungsgehäuse (20) mindestens eine Bremseinheit (32) angeordnet ist, die zur Anlage an Flächen (44, 42) der Tischplatte (16) bestimmte Bremselemente (54. 62) und einen Verstellmechanismus (64, 70) für die Bremselemente (54, 62) hat.

13. Lagerfläche nach Anspruch 12, **dadurch gekennzeichnet, daß** die Bremselemente (54, 62) und der Verstellmechanismus (64, 70) an einem Bremsenträger (50) angeordnet sind, der mittels Rollen (52) an einer oberen Führungsfläche (44) der Tischplatte (16) gelagert ist und gegen eine Bewegung in Längsrichtung der Tischplatte (16) an dem ersten Führungsgehäuse (20) gesichert ist.

14. Lagerfläche nach einem der Ansprüche 1 bis 14, **dadurch gekennzeichnet, daß** die Führungseinheit (18) ein zweites Führungsgehäuse (22) hat, an dem Kopplungs- und Führungselemente für die Kopplung mit der Tragsäule (14) und eine Querbewegung der Lagerflächen (10) relativ zur Tragsäufe (14) vorgesehen sind.

## Claims

1. A support surface for a medical examination table for carrying out X-ray examinations and surgical interventions with an intra-operative use of X-ray devices, including a table plate (16), which consists entirely of X-ray transmitting material and has guide surfaces (42, 44; 88) with which it bears against guide elements (38, 40) making possible a shifting of the table plate relative to a support column (14) of the examination table, the table plate (16) having two edge strips (26) forming its longitudinal edges as well as a middle section (24) which is thicker than the edge strips (26), **characterized in that** the support surface (10) has a guide unit (18) with a first guide housing (20) couplable with the support column (14) of the examination table, in which guide housing (20) the guide elements (38, 40) are arranged and in which the table plate (16) is slidably guided in its longitudinal direction, and **in that** at least the upper guide surfaces (44) are formed on the upper side of the edge strips (26).

2. The support surface according to claim 1, **characterized in that** the middle section (24) has a trapezoidal shape in cross section transversely to its longitudinal direction, with the underside of the middle section (24) being smaller than its upper side.

3. The support surface according to claim 1 or 2, **characterized in that** the guide elements are upper and lower support or guide rollers (40, 38) which simultaneously directly bear against upper and lower guide surfaces (44, 42), respectively, of the table plate (16).

4. The support surface according to one of the claims 1 to 3, **characterized in that** the lower guide surfaces (44, 42) are formed along the two longitudinal edges (26) of the table plate (16) on the underside thereof.

5. The support surface according to claim 3 or 4, **characterized in that** several lower and upper guide rollers (38, 40) are included in a roller block (30) and are supported on a roller carrier (36) which is pivotally supported on the guide housing (20) for movement about an axis (46) extending transversely to the longitudinal direction and parallel to the table plate (16), with at least two roller blocks (30) being arranged on the guide housing (20) on each longitudinal side of the support surface (10).

6. The support surface according to one of the claims 1 to 5, **characterized in that** at least one friction drive (32) engaging an edge strip (26) of the table plate (16) is provided in the guide housing (20).

7. The support surface according to claim 6, **characterized in that** the friction drive (32) includes a drive roller (76) coupled with a motor and two friction rollers (78) which, on the one hand, are in permanent engagement with the drive roller (76) and, on the other hand, are biased against a guide surface (42) of the table plate (16).

8. The support surface according to one of the claims 1 to 3, **characterized in that** the upper guide surfaces (44) are formed on the edge strips (26) and **in that** on the underside of the middle section (24) at least one lower guide surface (88) is formed.

9. The support surface according to claim 8, **characterized In that** the guide roller (38) which bears against the at least one lower guide surface (88) extends transversely over the entire width of the table plate (16).

10. The support surface according to claim 8 or 9, **characterized in that** the upper guide rollers (40) are formed as track rollers with a lateral guide (104) for the table plate (16).

11. The support surface according to one of the claims 8 to 10, **characterized in that** the lower guide rollers (38) are drivable by a motor (90).

12. The support surface according to one of the claims 1 to 11, **characterized in that** in the guide housing (20) is arranged at least one brake unit (32) having brake elements (54, 62) for engagement with the surfaces (44, 42) of the table plate (16) and a positioning mechanism (64, 70) for the brake elements (54, 62).

13. The support surface according to claim 12, **characterized in that** the brake elements (54, 62) and the positioning mechanism (64, 70) are arranged on a brake carrier (50), which brake carrier is supported by means of rollers (52) on an upper guide surface (44) of the table plate (16) and is secured on the first guide housing (20) against movement in the longitudinal direction of the table plate (16).

14. The support surface according to one of the claims 1 to 14, **characterized in that** the guide unit (18) has a second guide housing (22) on which are provided coupling and guide elements for coupling with the support column (14) and for a lateral movement of the support surface (10) relative to the support column (14).

## Revendications

1. Surface porteuse pour table d'examen médical qui est destinée à effectuer des examens radiologiques et des interventions chirurgicales avec utilisation d'appareils radiologiques pendant l'opération, comportant une plaque de table (16) qui est entièrement en matériau transparent aux rayons X et des surfaces de guidage (42, 44; 88) à l'aide desquelles elle porte contre des éléments de guidage (38, 40) pour un déplacement par rapport à une colonne de support (14) de la table d'examen, la plaque de table (16) comportant deux bandes de bord (26) constituant ses bords longitudinaux ainsi qu'une portion médiane (24) plus épaisse que les bandes de bord (26), **caractérisée en ce que** la surface porteuse (10) comporte une unité de guidage (18) pouvant être couplée à la colonne de support (14) de la table d'examen avec un premier boîtier de guidage (20) dans lequel sont placés les éléments de guidage (38, 40) et dans lequel la plaque de table (16) est guidée en translation dans sa direction longitudinale, et **en ce que** au moins les surfaces de guidage supérieures (44) sont réalisées du côté supérieur des bandes de bord (26).

2. Surface porteuse selon la revendication 1, **caractérisée en ce que** la portion médiane (24) a une section trapézoïdale transversalement à sa direction longitudinale, le côté inférieur de la portion médiane (24) étant plus petit que son côté supérieur.

3. Surface porteuse selon la revendication 1 ou 2, **caractérisée en ce que** les éléments de guidage sont des rouleaux d'appui ou de guidage supérieurs et inférieurs (40, 38) qui portent simultanément directement contre des surfaces de guidage inférieures respectivement supérieures (44, 42) de la plaque de table (16).

4. Surface porteuse selon l'une des revendications 1 à 3, **caractérisée en ce que** les surfaces de guidage inférieures (44,42) sont conformées le long des deux bords longitudinaux (26) de la plaque de table (16) du côté inférieur de celle-ci.

5. Surface porteuse selon la revendication 3 ou 4, **caractérisée en ce que** plusieurs rouleaux de guidage inférieurs et supérieurs (38, 40) sont assemblés en un bloc de rouleaux (30) et sont montés sur un support de rouleaux (36) qui est monté sur le boîtier de guidage (20) de façon à pouvoir pivoter autour d' un axe (46) dirigé transversalement à la direction longitudinale et parallèlement à la plaque de table (16), au moins deux blocs de rouleaux (30) étant disposés sur le boîtier de guidage (20) de chaque côté longitudinal de la surface porteuse (10).

6. Surface porteuse selon l'une des revendications 1 à 5, **caractérisée en ce qu'**il est prévu dans le boîtier de guidage (20) au moins un entraînement à friction (32) s'engageant avec une bande de bordure (26) de la plaque de table (16).

7. Surface porteuse selon la revendication 6, **caractérisée en ce que** l'entraînement à friction (32) comporte un rouleau d'entraînement (76) couplé à un moteur et deux rouleaux à friction (78) qui sont d'une part en engagement permanent avec le rouleau d' entraînement (76) et qui 'sont contraints d'autre part contre une surface de guidage (42) de la plaque de table (16).

8. Surface porteuse selon l'une des revendications 1 à 3, **caractérisée en ce que** les surfaces de guidage supérieures (44) sont réalisées au niveau des bandes de bord (26) et **en ce que** au moins une surface de guidage inférieure (88) est réalisée du côté inférieur de la portion médiane (24).

9. Surface porteuse selon la revendication 8, **caractérisée en ce que** le rouleau de guidage (38) portant contre l'au moins une surface de guidage inférieure (88) s'étend transversalement sur toute la largeur de la plaque de table (16).

10. Surface porteuse selon la revendication 8 ou 9, **caractérisée en ce que** les rouleaux de guidage supérieurs (40) sont réalisés en rouleaux de conduite comportant un guidage latéral (104) destiné à la plaque de table (16).

11. Surface porteuse selon l'une des revendications 8 à 10, **caractérisée en ce que** les rouleaux de guidage inférieurs (38) peuvent être entraînés par un moteur (90).

12. Surface porteuse selon l'une des revendications 1 à 11, **caractérisée en ce que** au moins une unité de freinage (32) est placée dans le boîtier de guidage (20) laquelle comporte des éléments de freinage (54, 62) destinés à porter contre des surfaces (44, 42) de la plaque de table (16) et un mécanisme de réglage (64, 70) destiné aux éléments de freinage (54, 62).

13. Surface porteuse selon la revendication 12, **caractérisée en ce que** les éléments de freinage (54, 62) et le mécanisme de réglage (64, 70) sont placés sur un support de freinage (50) qui est monté au moyen de rouleaux (52) sur une surface de guidage supérieure (44) de la plaque de table (16) et qui est empêché de se déplacer dans la direction longitudinale de la plaque de table (16) sur le premier boîtier de guidage (20).

14. Surface porteuse selon l'une des revendications 1 à 14, **caractérisée en ce que** l'unité de guidage (18) comporte un deuxième boîtier de guidage (22) au niveau duquel il est prévu des éléments de guidage et de couplage pour le couplage avec la colonne de support (14) et pour un mouvement transversal des surfaces porteuses (10) par rapport à la colonne de support (14).
